# EUROPÄISCHE PATENTSCHRIFT

(11) **EP 1 984 032 B1**
(45) Veröffentlichungstag und Bekanntmachung des Hinweises auf die Patenterteilung: **27.01.2016**
(21) Anmeldenummer: 07702422.2
(22) Anmeldetag: 02.02.2007
(51) Int. Cl.: A61L 24/10, C09J 189/00

(54) **NEUE KLEBSTOFFE FÜR MEDIZINISCHE ANWENDUNGEN**
NOVEL ADHESIVES FOR MEDICAL APPLICATIONS
NOUVELLES COLLES AUX FINS D'APPLICATIONS MÉDICALES

(30) Priorität: 09.02.2006 DE 102006006904
(43) Veröffentlichungstag der Anmeldung: 29.10.2008
(60) Teilanmeldung: 15202504.5
(73) Patentinhaber: Aesculap AG, 78532 Tuttlingen (DE)
(72) Erfinder: BEHREND, Detlef, 18119 Warnemünde (DE); SCHMITZ, Klaus-Peter, 18119 Warnemünde (DE); STERNBERG, Katrin, 18055 Rostock (DE); SCHMOHL, Kathleen, 18055 Rostock (DE); KRAMER, Sven, 18107 Rostock (DE); NIES, Berthold, 64407 Fränkisch-Crumbach (DE)
(74) Vertreter: UEXKÜLL & STOLBERG
(86) Internationale Anmeldenummer: PCT/DE2007/000192
(87) Internationale Veröffentlichungsnummer: WO 2007/090373

(56) Entgegenhaltungen:
- WO-A-94/28937
- WO-A-96/03159
- DE-A1- 19 958 526
- JOBMANN M ET AL: "End-group functionalized polylactides: a potential protein carrier" JOURNAL OF CONTROLLED RELEASE, ELSEVIER, AMSTERDAM, NL, Bd. 41, Nr. 1, 1. August 1996 (1996-08-01), Seite S8, XP004037551 ISSN: 0168-3659

## Beschreibung

Die Erfindung betrifft Kleber, vor allem medizinische Kleber, die zum Kleben von Hart- und Weichgewebe geeignet sind. Es wird ein Kombinationspräparat nach Anspruch 1 aus Peptiden, endständig funktionalisierten Oligolactonen und, optional, Katalysatoren einer Vernetzungsreaktion zwischen diesen Komponenten offenbart. Der Begriff Oligolacton umfasst in diesem Zusammenhang übergreifend "innere Ester" von Hydroxycarbonsäuren und somit auch Oligoglykolide, Oligolactide und deren Copolymere.

### Stand der Technik

Als Wunden werden Kontinuitätsunterbrechungen von Körperoberflächen oder Körperinnenflächen bezeichnet. Die Heilung verläuft in mehreren fließend ineinander übergehenden Phasen (Proliferation, Organisation, Epithelisation). Das Wundmanagement bei akuten Wunden hat bei Nicht-Risiko-Patienten einen medizinisch hohen Stand erreicht.

Schwierig ist auch heute noch die akute Versorgung stark blutender Wunden. Hier steht die Gefahr der Verblutung im Vordergrund. Wenn Wundkleber eingesetzt werden sollen, besteht die Gefahr, dass Klebekomponenten mit dem Blutstrom weggespült werden und die Verklebung an einem unerwünschten Ort eintritt. Damit wird die Thrombose-Gefahr erhöht.

Eine Blutstillung kann nach dem Stand der Technik erreicht werden, z.B. durch
a) Proteindenaturierung z.B. durch Metallsalze
b) mikroporöse Biopolymere bzw. Biopolymere mit großer innerer Oberfläche. Diese absorbieren Wasser, dadurch kommt es zu einer Konzentration der Blutzellen sowie der Gerinnungsfaktoren. Als besonders vorteilhaft haben sich Polysaccharide erwiesen, die aus Poly-N-Acetyl-Glucosaminen oder Chitosan bestehen. Diese sind z.B. im Skelett von Insekten, aber auch in Algen enthalten. Biopolymere wie Gelatine, Kollagen, Fibrinschwämme oder oxidierte Cellulose haben eine große innere Oberfläche. Aufgrund der Absorption an diese große Fläche kommt es zu einer Konzentration der Gerinnungsfaktoren.
c) Enzyme, insbesondere durch Thrombin oder proteolytische Enzyme mit thrombinähnlicher Wirkung mit oder ohne Zusatz von Calcium
d) hochkonzentrierte Gerinnungsfaktoren
e) Kombinationen von c) und d)
f) Calzium-Alginate
g) alumosilikatische Syntheseprodukte mit Mikroporenstruktur.

Die bisher zur Blutstillung eingesetzten Syntheseprodukte sind vor allem Alkali- und Erdalkalialuminiumsilicate unterschiedlicher Zusammensetzung.

Auf der Basis von synthetisierten und bei über 400°C vollständig dehydratisierten Erdalkalialuminiumsilicaten wurde von der Firma Z-Medica, U.S.A., für die Sofortbehandlung von Kriegsverletzungen und zur Notversorgung ein Syntheseprodukt einwickelt. Durch eine starke exotherme Reaktion wird dem Blut Wasser entzogen und es kommt so zur Blutstillung. Das Syntheseprodukt wird direkt in Granulatform auf die Wunde appliziert. Es wird unter der Bezeichnung "Quikclot" vertrieben. Nachteilig wurde bei diesem Produkt beschrieben, dass durch die exotherme Reaktion (Temperaturen bis 60 °C) Schädigungen des angrenzenden Gewebes auftreten (Journal of Trauma 54 (2003) 6, 1077 - 1082).

Synthetische Lithiumalumosilikate (EP 1176991 A1, WO 00/69480) wurden speziell für die Wundversorgung entwickelt und werden unter dem Handelsnamen CERDAC vertrieben. Im Falle von CERDAC wird unter Aufwendung hoher Temperaturen (> 1000 °C) ein mikroporosiertes keramisches Produkt hergestellt, um eine optimale Wundversorgung zu erreichen. Abgesehen von der z.T. aufwändigen Herstellung ist die Kapillarkraftwirkung zu gering, um allen Anforderungen zu genügen.

Ein großes Problem besteht weiterhin in einer möglichen Chronifizierung der Wunden, insbesondere bei Risikopatienten. Diese kann bisher nicht sicher vermieden werden, und die Behandlung solcher Wunden ist nach wie vor sehr schwierig. Hier genügt die Wundversorgung über Wundverbände im Allgemeinen nicht mehr. Mit der Chronifizierung ist oft eine Keimbesiedlung der Wunde verbunden.

Allein in Deutschland leiden 4 Millionen Patienten an chronisch offenen Wunden. Die jährlichen Kosten betragen 1,7 bis 3,2 Milliarden Euro. Für die Versorgung chronischer Wunden wurde bisher keine befriedigende Lösung erreicht.

Insgesamt kann festgestellt werden, dass sowohl für eine schnelle Verschließung, die Versorgung von stark blutenden als auch vor allem für die Versorgung chronischer Wunden bisher noch keine in jeder Hinsicht befriedigenden Lösungen erreicht wurden.

Die Entwicklung neuer Klebstoffe, die für diese Problemfälle eingesetzt werden können, hat in den letzten Jahren stark an Bedeutung gewonnen. Bisher eingesetzte Wundkleber auf AcrylatBasis können nur auf oberflächlichen Wunden eingesetzt werden, da die Acrylate biologisch nicht abgebaut werden und eine toxische Gefährdung bedeuten können. Ein Augenmerk liegt deshalb vor allem auf der biologischen Abbaubarkeit der eingesetzten Materialien. Aus diesem Grund spielen für die Klebstoffherstellung beispielsweise Polymere pflanzlichen bzw. tierischen Ursprungs eine große Rolle. Muscheln sind z. B. in der Lage, unter Wasser besonders feste Verbindungen mit verschiedenen Trägersubstraten einzugehen. Grund dafür ist die Bildung/Ausscheidung von MAPs (mussel adhesive proteins). MAPs bestehen aus einer Vielzahl von Proteinen, die je nach Muschelart variieren kann.

Im Stand der Technik sind Kleber auf Basis von MAP bekannt. MAP werden dabei, z.B. durch Catecholoxidase, direkt miteinander verknüpft (US 5,015,677). Auch die Vernetzung von polyphenolischen MAP durch Polymere wie Poly(alkylenoxid) oder Polyethylenglykol (PEG) ist bekannt (WO 94/28937).

Jobman et al., J. Contr. Rel. 41(1), 1996, S8 beschreibt eine Kombination von Bovinem Serumalbumin (BSA) mit endständig funktionalisiertem Oligolacton.

WO 96/03159 offenbart Klebstoffe mit reaktionsfähigen Polymeren auf Poly(ethylenglykol) (PEG)-Basis mit BSA.

DE 199 58 526 betrifft einen Polyurethan-Klebstoff.

Die Aufgabe, die durch die Erfindung gelöst werden sollte, bestand darin, alternative, als Gewebekleber besonders geeignete Kombinationspräparate und entsprechende Verfahren zum Kleben von Gewebe bereitzustellen. Dabei entstanden Produkte, die effiziente Klebstoffeigenschaften für Hart- und Weichgewebe aufweisen.

Erfindungsgemäß wird diese Aufgabe durch ein Kombinationspräparat, Verfahren und Verwendungen gemäß den Merkmalen der Patentansprüche gelöst.

So wird insbesondere eine Kombination nach Anspruch 1 bereitgestellt, die
a) ein Peptid, das mindestens zwei reaktive Gruppen umfasst, ausgewählt aus einer Gruppe bestehend aus Hydroxyl und/oder Amino, und
b) ein endständig funktionalisiertes Oligolacton
umfasst.

Im Rahmen der vorliegenden Erfindung wird als Peptid ein Oligopeptid (2 bis ca. 100 Aminosäuren Länge) bezeichnet. Bevorzugt weist das Peptid eine Länge von 4-100 Aminosäuren, insbesondere 10-20 Aminosäuren auf. Das Peptid kann ein Molekulargewicht von etwa 1 kDa bis etwa 50 kDa, insbesondere etwa 2 kDa bis etwa 50 kDa oder etwa 5 kDa bis etwa 20 kDa aufweisen. Es kann modifiziert bzw. substituiert sein, z.B. glykosyliert. Neben den üblichen proteinogenen Aminosäuren können auch modifizierte bzw. untypische Aminosäuren wie Hydoxylysin in dem Oligopeptid enthalten sein. Der Einsatz von D-Aminosäuren anstelle von L-Aminosäuren oder zusätzlich dazu ist möglich und verlangsamt den Abbau des Peptids.

Selbstverständlich können auch mehrere verschiedene derartige Peptide in der erfindungsgemäßen Kombination enthalten sein. Diese Variante ist in der Folge nicht gesondert erwähnt, jedoch stets mit umfasst.

Die reaktiven Aminogruppen können primäre oder sekundäre Aminogruppen sein. Mindestens eine der reaktiven Aminogruppen des Peptids ist bevorzugt Bestandteil einer Diaminosäure. Bevorzugt umfasst das Peptid daher mindestens eine Diaminosäure, bevorzugt mindestens 2, 3, 4, 5 oder mehr Diaminosäuren. Bevorzugt werden Lysin-haltige Peptide (bzw. Oligopeptide oder Eiweiße) eingesetzt.

Auch andere Aminosäuren, wie Arginin, Asparagin, Glutamin oder Histidin, tragen reaktive Aminogruppen, die mit den Oligolactonen reagieren können.

Die reaktiven Aminogruppen, insbesondere die durch die Diaminosäure bereitgestellten Aminogruppen, sind für die Vernetzungsreaktion zwischen Peptid und Oligolacton besonders geeignet. Auch Hydroxylgruppen in dem Peptid können zu der Vernetzungsreaktion beitragen. Bevorzugt umfasst das Peptid daher mindestens eine Aminosäure mit einer Hydroxylgruppe, also insbesondere Serin, Threonin oder Tyrosin. Auch Hydroxylysin oder polyphenolische Aminosäurebausteine, wie sie in den MAPs vorliegen, können in den erfindungsgemäß eingesetzten Peptiden vorkommen. Ein Vorteil der vorliegenden Erfindung ist jedoch, dass die Anwesenheit dieser spezifischen Aminosäurebausteine und damit die Verwendung von MAPs nicht notwendig ist. Die verwendeten Peptide können daher z.B. ohne weiteres rekombinant hergestellt werden.

Die durch Reaktion der Oligolactone mit dem Peptid entstehende Vernetzung hängt wesentlich davon ab, wie hoch der Anteil der zur Verfügung stehenden reaktiven Gruppen in dem Peptid ist. Klebeeigenschaften können ab einem molaren Anteil an Aminosäuren des Peptids, die eine freie Aminogruppe (z.B. Diaminosäuren wie Lysin) und/oder Hydroxylgruppe tragen, von mindestens 10 % erreicht werden. Bevorzugt liegt der Anteil dieser Aminosäuren jedoch höher, bei mindestens 20%, mindestens 30%, mindestens 40 oder mindestens 50% oder sogar bei mindestens 80 bis 100%. Diese Kriterien werden von natürlich vorkommenden Peptiden und Proteinen erfüllt. Besonders geeignet sind z.B. MAPs.

Es können jedoch auch besonders geeignete kürzere, leicht künstlich herstellbare Peptide eingesetzt werden. In einer besonders bevorzugten Ausführungsform der Erfindung sind etwa 50% der Aminosäuren des Peptids Lysin und/oder etwa 50% der Aminosäuren des Peptids Tyrosin. Diese können z.B. als sich wiederholende Dipeptid-Einheit angeordnet sein. Auch eine andere Abfolge oder die Aufnahme weiterer Aminosäuren, insbesondere von Arginin, Asparagin, Glutamin oder Histidin (anstelle von Lysin oder zusätzlich), Serin oder Threonin (anstelle von Tyrosin oder zusätzlich) ist jedoch möglich. Eine Länge des Peptids von ca. 10-20 Aminosäuren ist besonders bevorzugt. Hervorragende Ergebnisse konnten etwa mit Peptiden von 10-20 Aminosäuren Länge, die aus sich wiederholenden Dipeptideinheiten von Lysin und Tyrosin bestanden ([Lys-Tyr]ₙ oder [Tyr-Lys]ₙ, n=5 bis n=10), erzielt werden.

Das im Rahmen der vorliegenden Erfindung eingesetzte Vernetzungsmittel ist ein funktionalisiertes Oligolacton. Als Oligolactone werden im Rahmen der Erfindung "innere Ester" von Hydroxycarbonsäuren und somit im erweiterten Sinne auch Oligoglykolide, Oligolactide und deren Copolymere bezeichnet, die, wie im Stand der Technik bekannt, durch ringöffnende Polymerisation hergestellt werden können. Unter endständig funktionalisierten Oligolactonen werden Polymere von Hydroxycarbonsäuren mit einem zentralen mindestens zweiwertigen Alkohol verstanden, wobei die Hydroxylgruppen des Alkohols mit Lactonen bzw. Lactiden oder Glykoliden durch Ringöffnung verestert sind und wobei die freien Enden der Polyester reaktionsfähige funktionelle Gruppen tragen. Ein Beispiel ist in Reaktionsschema 1 dargestellt.

Es wird ein endständig funktionalisiertes Oligolacton verwendet. Bevorzugt weist das Oligolacton endständige Isocyanatgruppen auf.

Auch eine Funktionalisierung mit Aldehyd- oder Epoxidgruppen ist möglich, und das erhaltene Produkt kann in der erfindungsgemäßen Kombination eine Klebewirkung erzielen. Es stellte sich überraschenderweise heraus, dass durch Funktionalisierung der Oligolactone mit Isocyanat sowohl eine erhöhte Festigkeit des Polymerisats als auch eine größere Haftfestigkeit erreicht wurde.

Das Oligolacton ist ein Ethylenglycol-Oligolactid (EOL), wie es in Reaktionsschema 1 dargestellt ist. Dabei ist n = 1 (bei Umsetzung von 1 Teil Ethylenglykol mit 2 Teilen Lactid, d. h. 1/2 ist M = 350,3 g/mol). Mit zunehmender Anzahl der Einheiten steigt die Viskosität des Oligolactons und der im Rahmen einer Reaktion mit den Peptiden erhaltenen Kleber an. Eine optimale Handhabbarkeit wurde mit Oligolactonen (n=1) erzielt, da dadurch eine gute Verarbeitung bei einem geringen Lösungsmittelanteil gewährleistet war. Außerdem wurden weitere Ethylenglykol-Derivate wie Ethylenglycololigoglycolid (EOG 1/2, M = 294,2 g/mol) mit endständigen Isocyanatgruppen versehen. Auch mit diesen Derivaten wurden gute Klebeeigenschaften erhalten.

Analog ist auch die Verwendung weiterer Oligolactone, etwa auf der Basis von Polymerisationsprodukten von Glycerol und Pentaerythrit möglich. So entstehen beispielsweise bei der Umsetzung von Pentaerythritooligolactid (POL 1/4, M = 712,6 g/mol), Glycerololigolactid (GOL 1/0,5, M = 164,1 g/mol) und Glycerololigolactidco-glycolid (GOLG 1/1/3, M = 584,4 g/mol) mit Hexamethylendiisocyanat (HDI) folgende Produkte mit endständigen Isocyanatgruppen: POL-NCO, EOG-NCO und GOLG-NCO. 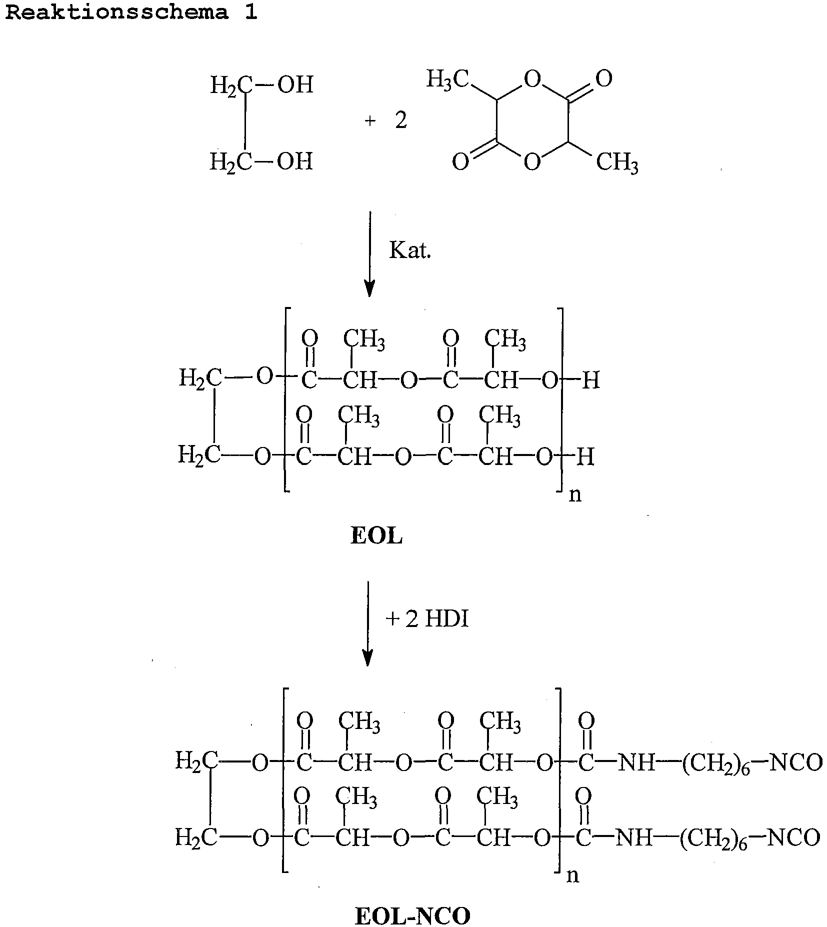 Selbstverständlich können auch mehrere verschiedene Oligolactone in der erfindungsgemäßen Kombination enthalten sein. Diese Variante ist in der Folge nicht gesondert erwähnt, jedoch stets mit umfasst.

Als Katalysator für die Herstellung der Oligolactone können organische Metallverbindungen, etwa Zink- oder Zinnverbindungen eingesetzt werden (H. R. Kricheldorf, H. R.,Kreiser-Saunders, I., Boettcher, C.:" Polylactones: 31. Sn(II)octoate-initiated polymerization of L-lactide: a mechanistic study",in: Polymer Vol. 36 No. 6, pp. 1253-1259, 1995; Kreiser-Saunders, I., Kricheldorf, H. R.:". Polylactones: 39a. Zn lactate-catalyzed copolymerisation of L-lactide with glycolide or ε-caprolactone", in: Macromol. Chem. Phys. 199, 1081-1087, 1998; Kricheldorf, H. R., Kreiser-Saunders, I., Damrau, D. O:" Resorbable Initiators for Polymerization of Lactones", in: Macromol. Symp. 144, 269-276, 1999; Kricheldorf, H. R., Kreiser-Saunders, I.:"Polylactides-Synthesis, Characterization and Medical Application", in: 103, 85-102, 1996).

Beispiele sind Zink- bzw. Zinn-(II)-salze von organischen Carbonsäuren, z.B. Zink-(II)-octoat, Zink-(II)-ethyl-hexoat, Zinn-(II)-acetat, Zinn-(II)-octoat, Zinn-(II)-ethylhexoat und Zinn-(II)-laurat, und Dialkylzinn(IV)-salze von organischen Carbonsäuren, z.B. Dibutyl-zinndiacetat, Dibutylzinn-dilaurat, Dibutylzinnmaleiat und Dioctylzinn-diacetat. Weiterhin können auch Eisen (III)-salze verwendet werden, wie z.B. Eisen(III)-chlorid.

Um die Bioverträglichkeit zu erhöhen, werden die Katalysatoren dieser Reaktion vor der Verwendung der Oligolactone bevorzugt entfernt, so dass sie in Mengen von unter 0,1%, bevorzugt von unter 0,01% vorliegen. Dazu sind im Stand der Technik Verfahren bekannt (EP1497340, EP1221454).

Alternativ oder zusätzlich werden, vor allem zur Herstellung von Medizinprodukten, die besser biologisch verträglichen Zinkverbindungen oder Eisenverbindungen eingesetzt.

Die Oligolactone können mit Diisocyanaten, z.B. mit Hexamethylendiisocyanat umgesetzt werden, um Oligolactone mit endständigen Isocyanatgruppen herzustellen. Bevorzugt werden für die Umsetzung aliphatische Isocyanate verwendet, da sich aus aromatischen Diisocyanaten kanzerogene Diamine bilden können. Bei einer stöchiometrischen Umsetzung ist eine Aufreinigung nicht nötig. Gegebenenfalls kann jedoch eine Aufreinigung z.B. über Destillation durchgeführt werden.

Sofern eine schnelle Verklebung gewünscht ist, wird im Rahmen der erfindungsgemäßen Kombination ferner als Komponente c) ein Katalysator der Reaktion zwischen Peptid und Oligolacton verwendet. Dies ist insbesondere für medizinische Zwecke sinnvoll. Insbesondere beschleunigt der Katalysator die Reaktion der Hydroxylgruppen und/oder freie Aminogruppen (bevorzugt Diaminosäuren) enthaltenden Peptide mit den Isocyanatgruppen der Oligolactone stark.

Katalysatoren beschleunigen die Reaktionsgeschwindigkeit einer chemischen Reaktion, ohne dabei selbst verbraucht zu werden. Eine solche starke Beschleunigung (etwa um den Faktor 10-100) ist, abhängig auch von den Ausgangsstoffen, insbesondere beim medizinischen Einsatz, z.B. beim Verkleben von blutenden Wunden sinnvoll. Es kann jedoch auch eine langsamere Reaktionsgeschwindigkeit bevorzugt sein, um, etwa beim Verkleben von Knochen, zunächst eine Einpassung mit der Möglichkeit einer späteren Korrektur zu erlauben. Insbesondere beim Einsatz von Oligopeptiden mit einem hohen Anteil (mindestens 30%, bevorzugt mindestens 50 %) von Aminosäuren mit reaktiven Aminogruppen, vorzugsweise Diaminosäuren, ist die Reaktionsgeschwindigkeit auch ohne Katalysator so hoch, dass dieser für eine schnelle Verklebung nicht notwendig ist. Mit Katalysator wird mit diesen Peptiden eine besonders schnelle Verklebung erreicht.

Mit den erfindungsgemäßen Kombinationen kann bevorzugt eine feste Verklebung im Zeitrahmen von etwa 30 Sekunden bis 15 Minuten, besser etwa 1 bis 5 Minuten erfolgen. In dieser Zeit muss jedoch nicht eine vollständige Reaktion aller Komponenten, d. h. eine vollständige Bulkpolymerisation, erfolgt sein, sondern es reicht aus, wenn eine Festigkeit erreicht wird, bei der die verklebten Substrate fixiert sind, z.B. Wundränder aneinander haften, ohne sich zu verschieben. Die Reaktion läuft mit der erfindungsgemäßen Kombination mit Katalysator in einem Zeitraum von ca. 3-60 Minuten, vorzugsweise ca. 3-10 Minuten im wesentlichen vollständig ab, ohne Katalysator beträgt der Zeitraum für einen im wesentlichen vollständigen Ablauf der Reaktion ca. 30 Minuten bis zu mehreren Tagen, vorzugsweise ca. 30-120 Minuten.

Als Katalysator kann etwa ein basisches Amin, ein Amidin, vorteilhafterweise 2,3-Dimethyl-3,4,5,6-tetrahydropyrimidin, ein tertiäres Amin, vorteilhafterweise Triethylamin, Tributylamin, Dimethylbenzylamin, N-Methyl-, N-Ethyl-,N-Cyclohexylmorpholin, N,N,N',N'Tetramethyl-ethylendiamin, N,N,N',N'-Diaminoethylether, Bis-(dimethylaminopropyl)harnstoff, Dimethylpiperazin, 1,2-Dimethylimidazol, 1-Aza-bicyclo-(3,3,0)-octan und vorzugsweise 1,4-Diaza-bicyclo-(2,2,2)-octan und/oder ein Alkanolamin, wie Triethanolamin, Triisopropanolamin, N-Methyl- und N-Ethyl-diethanolamin und Di-methylethanolamin, vorzugsweise 1,4-Diaza[2.2.2]bicyclo-octan (Dabco), eingesetzt werden. Bevorzugt ist der Katalysator 1,4-Diaza[2.2.2]bicyclooctan (Dabco).

In einer bevorzugten Ausführungsform umfasst die erfindungsgemäße Kombination nach Anspruch 1
a) ein Oligopeptid, das mindestens eine, vorzugsweise zwei oder mehr Diaminosäuren und/oder Hydroxylgruppen enthält,
b) ein mit Isocyanaten endständig funktionalisiertes Oligolacton und
c) einen Katalysator für die Reaktion des Oligopeptids mit dem Oligolacton.

In einer weiteren Ausführungsform kann ferner zusätzlich
d) Kettenverlängerer zur Bulkpolymerisation (ausgewählt aus einer Gruppe umfassend Diole, Diamine, Oligolactone wie EOL, EOG, GOL, GOLG, POL)
in der erfindungsgemäßen Kombination enthalten sein. Die Anwesenheit eines solchen zusätzlichen Kettenverlängerers ist jedoch nicht zwingend notwendig.

Insbesondere sind folgende Einzelkomponenten in der erfindungsgemäßen Kombination enthalten:
a) Oligopeptide, die mindestens eine Diaminosäure enthalten und
b) endständig funktionalisierte Oligolactone und
c) Katalysatoren, welche die Reaktion der Hydroxylgruppen enthaltenden Ausgangskomponenten mit den Isocyanaten stark beschleunigen.

Die erfindungsgemäßen Kombinationen können zusätzlich Kollagen enthalten oder auf einer Kollagenmatrix aufgebracht werden. Bevorzugt dienen sie zur Fixierung von Kollagen. Die Komponenten der Erfindung sowie weitere Zusatzstoffe können in unterschiedlichen Schichten auf eine Kollagenmatrix aufgebracht werden. Weiterhin können auch Kollagenhydrolysate, wie z. B. Gelatine, und Proteine wie Albumin in der erfindungsgemäßen Kombination enthalten sein.

Im Allgemeinen liegen die einzelnen Komponenten der Kombination getrennt voneinander vor, insbesondere liegen die Komponenten a und b getrennt voneinander vor. Es kann jedoch auch eine Vormischung von Komponenten erfolgen, die die Verwendung vorbereitet, beispielsweise können Peptid und Katalysator bereits gemischt sein. Vorteilhafterweise werden Oligolacton und Peptid erst dann gemischt, wenn die Reaktion zwischen beiden erwünscht ist. Wenn jedoch der Katalysator erst direkt vor der Anwendung hinzugefügt wird, kann auch eine Mischung, die sowohl Peptid als auch Oligolacton umfasst, noch in einem gewissen Rahmen lagerfähig sein.

In dem Produkt können eine oder mehrere Komponenten - gemeinsam oder getrennt - in einem oder mehreren Lösungsmitteln aufgenommen sein. Für medizinische Anwendungen ist das Lösungsmittel in geringen Konzentrationen biologisch verträglich. Es kann ein organisches Lösungsmittel wie DMSO sein.

In einer Ausführungsform wird als Lösungsmittel eine Mischung von DMSO und Wasser verwendet, wobei der Anteil von Wasser etwa 5-20% beträgt, bevorzugt etwa 8-12% oder etwa 10%. Insbesondere wird das Peptid (und gegebenenfalls der Katalysator) in dem wässrigen bzw. wasserhaltigen Lösungsmittel gelöst. In diesem Fall entsteht bei der Reaktion aller Komponenten unter Bildung von CO₂ eine Verbindung mit schaumartiger, offenzelliger Struktur, wie sie aus der klassischen Polyurethan-Chemie bekannt ist. Bei Verwendung einer Mischung organischer mit wässrigen Lösungsmitteln wird bevorzugt weniger Peptid eingesetzt als mit organischen Lösungsmitteln, wie unten beschrieben. Die mit dieser Ausführungsform erhaltene schaumartige Struktur bildet bei medizinischen Anwendungen ein gutes Medium für eine Besiedlung mit Zellen. Gerade die Durchlässigkeit der entstehenden Verbindung trägt zur Biofunktionalität bei. Bei der Applikation als Schaum ist das Material besonders geeignet zum Wundverschluss z.B. bei großflächigen Sickerblutungen oder zum Auffüllen von Gewebedefekten, vorzugsweise Weichgewebedefekten.

Insbesondere für die Anwendung bei diffus blutenden Wunden ist auch eine Applikationsform der erfindungsgemäßen Kombination als Spray geeignet.

In einer Ausführungsform ist das Produkt bereits in einer Zweikomponentenspritze, bevorzugt bereits mit angesetztem Mischextruder, zur Anwendung vorbereitet, wobei die eine Komponente das Peptid und gegebenenfalls den Katalysator umfasst und die andere Komponente das Oligolacton. Dies erlaubt eine besonders exakte Dosierung und einfache Handhabung. Bevorzugt ist eine Doppelkammerspritze etwa vom Typ Mixpac (Mixpac Systems AG, Rotkreuz, Schweiz).

Alternativ kann die Kombination oder ihre Bestandteile direkt vor der Verwendung in einem Lösungsmittel aufgenommen werden.

Bevorzugt liegen die Bestandteile der Kombination schon in einem Mengenverhältnis vor, das für ihre Verwendung angemessen ist und ein umständliches Dosieren der Zutaten erspart. Der Anteil der Peptide an der Kombination kann z.B. etwa 20-70% (die Prozentangaben beziehen sich auf Gew.-%, bezogen auf die Gesamtmasse ohne Lösungsmittel), bevorzugt etwa 30-70%, etwa 40-70%, etwa 50-70% oder etwa 60-70% betragen. Der Anteil der Oligolactone kann z.B. etwa 30-80%, bevorzugt etwa 30-70%, etwa 30-60%, etwa 30-50% oder etwa 30-40% betragen. Der Anteil an Katalysator der Reaktion zwischen Peptid und Oligolacton beträgt etwa 0-10%, bevorzugt etwa 1-8%, etwa 2-6% oder etwa 3-5%.

Diese Mengenverhältnisse können in Bezug auf die gewünschte Anwendung angepasst werden. Eine anteilig größere Menge an Oligolacton führt dabei zu einem höheren Vernetzungsgrad. Ferner werden reaktive Amino- und Hydroxylgruppen von zu verklebenden Substraten verstärkt in die Vernetzung einbezogen, wenn ein geringerer Anteil an Peptid vorliegt. Eine Optimierung ist durch Versuche möglich.

Die Kombination kann weitere Zusatz- und Hilfsstoffe enthalten, z.B. Füllmittel wie Kollagen, Albumin, Hyaluronsäure oder ähnliches. Bevorzugt beträgt der Gesamtanteil von Peptid und Oligolacton etwa 50-100%, insbesondere etwa 50-90%. Auch die Wundheilung fördernde Substanzen, z.B. Wachstumsfaktoren, oder antibiotisch wirksame Substanzen können enthalten sein. Werden z.B. aminosäurehaltige antimikrobielle Wirkstoffe durch Einwirkung der erfindungsgemäßen Komponenten modifiziert und an Oberflächen fixiert, ergibt sich eine antimikrobielle Ausrüstung. Derartige Modifikationen sind vor allem zur Versorgung infizierter Wunden von Interesse.

Die erfindungsgemäße Kombination stellt einen neuartigen, wirksamen medizinischen Kleber dar. Bevorzugt ist die erfindungsgemäße Kombination ein Medizinprodukt, das zum Kleben von Organen, Gewebe, Zähnen und/oder Knochen geeignet ist. Eine Klassifizierung als pharmazeutisches Präparat ist jedoch, abhängig von nationalem Recht, ebenfalls möglich. Die Begriffe Medizinprodukt und pharmazeutisches Präparat sind für die Zwecke der Beschreibung der Erfindung austauschbar.

Bevorzugt liegen die Komponenten daher schon in einem für die Mischung geeigneten Verhältnis vor (s.o.) und sind steril verpackt, z.B. in einer Zweikomponentenspritze, bevorzugt mit aufgesetztem Mischextruder. Alternativ kann aber auch vorher die Mischung der Komponenten erfolgen, falls die Schnelligkeit der Verklebung im Vordergrund steht.

Eine Sterilisierung der erfindungsgemäßen Kombination bzw. ihrer einzelnen Komponenten kann vorteilhafterweise ohne Strukturveränderung erreicht werden, z.B. über Sterilfiltrierung von Lösungen. Bevorzugt ist jedoch eine Sterilisierung über GammaStrahlung, da diese bereits verpackt erfolgen kann und somit keine aseptische Abfüllung nötig ist. Es konnte gezeigt werden, dass bei Gamma-Sterilisation die Struktur der funktionalisierten Lactone erhalten bleibt.

Das Verkleben von Weich- und Hartgewebe erfolgt durch eine in situ Vernetzung von Oligopeptiden mit reaktiven, vorzugsweise mit Isocyanatgruppen endständig funktionalisierten Oligolactonen. Die Vernetzung erfolgt dabei über kovalente Bindungen, in die vorzugsweise die freien Aminogruppen der Kleberpeptide einbezogen werden. Dabei entsteht ein Wundverschluss, dessen Festigkeit signifikant höher ist als bei der Verwendung des herkömmlichen, klinisch etablierten Fibrinklebers. Die Haftung zum Gewebe wird darüber hinaus durch die Reaktion der endständigen reaktiven Gruppen mit den Gewebeproteinen erreicht. Zellvitalitätsuntersuchungen ergaben eine gute Biokompatibilität des Vernetzungsproduktes.

Die erfindungsgemäßen Kleber sind darüber hinaus weitaus leichter handhabbar. Besondere Anforderungen an die Lagerung, wie bei tiefgekühlten Fibrinklebern, bestehen nicht, d.h., die erfindungsgemäße Kombination kann nicht nur bei 4°C, sondern auch bei Raumtemperatur gelagert werden. Die erfindungsgemäßen Kleber sind eine Alternative sowohl zur Elektrokoagulation als auch zum Wundverschluss mit Nadel und Faden.

Durch die Erfindung wird auch eine Verbindung bereitgestellt, bei der die Komponenten der erfindungsgemäßen Kombination miteinander vermischt sind und die Peptide über die Oligolactone mit einander vernetzt sind. Bevorzugt sind auch reaktive Gruppen der zu verklebenden Substrate, wie Hydroxylgruppen und freie Aminogruppen, vernetzt.

Gegenstand der Erfindung ist daher auch ein Kleber und/oder ein Mittel zur Blutstillung, der/das die oben genannte Kombination enthält. Da eine schnelle Verklebung bei medizinischen Antworten oft sinnvoll ist, wird bevorzugt eine Kombination mit Katalysator eingesetzt. Eine Komponente dieses Klebers/Mittels sind die Oligopeptide, eine zweite Komponente endständige funktionalisierte Oligolactone, und als dritte Komponente fungieren Katalysatoren, die die Reaktion der Hydroxylgruppen enthaltenden Ausgangskomponenten mit den Isocyanaten stark beschleunigen.

Ein Vorteil dieses Klebers ist, dass die durch Reaktion der Kombination entstandene Verbindung biologisch abbaubar ist. Durch die Verknüpfung von Peptidsequenzen, die die für eine Klebewirkung relevanten Aminosäuren enthalten, entsteht eine sehr feste Verklebung, die aber im Verlauf des Heilungsprozesses resorbiert werden kann. Die anfängliche Härtungsphase, verbunden mit hohen Klebeigenschaften, und die zeitabhängig langsame Resorption im Körper bei fortgeschrittenen Heilungsprozessen sind von besonderem Vorteil.

Bevorzugt wird der Kleber innerhalb von etwa einem Jahr resorbiert (biologisch abgebaut), bevorzugt in einem Zeitraum von etwa 28 Tagen bis zu etwa 6 Monaten. Der Mechanismus der Resorption kann z.B. hydrolytisch oder enzymatisch sein. Insbesondere können die Oligopeptide gespalten und einzelne Fragmente abtransportiert und ausgeschieden werden. Alternativ können Fragmente oder Aminosäuren auch in das sich regenerierende Gewebe eingebaut werden.

In einer Ausführungsform stellt die Erfindung ein in vitro Verfahren zum Kleben bereit, bei dem man die Komponenten der erfindungsgemäßen Kombination bzw. des erfindungsgemäßen Medizinprodukts miteinander und mit mindestens einem, bevorzugt mindestens zwei, zu klebenden Substrat, das reaktive Hydroxylgruppen und/oder Aminogruppen aufweist, in Kontakt bringt. Bei dem Klebeverfahren erfolgt die Verbindung der Substrate bevorzugt durch eine Vernetzung von Hydroxylgruppen und/ oder Aminogruppen der Oligopeptide und der Substrate durch die Oligolactone. Die Erfindung betrifft auch die Verwendung der Medizinprodukte zum Wundverschluß, insbesondere zum Versiegeln von Wundoberflächen.

Dabei ist bevorzugt mindestens eins der Substrate ein Organ, Gewebe, Zahnkompartiment (Schmelz, Dentin und/oder Zement) und/oder Knochen. Vorzugsweise sind die Substrate Wundränder, die verklebt werden sollen. Alternativ kann es sich jedoch auch z.B. um miteinander zu verklebende anorganische oder organische Substrate handeln, die keine Gewebe sind, etwa Kunststoff- oder Keramikoberflächen. Eine medizinische Anwendung ist z.B. bei der Klebung von Implantaten denkbar.

Ebenfalls Gegenstand der Erfindung ist ein in einem wässrigen Milieu durchgeführtes Klebeverfahren unter Verwendung der oben genannten Kombination.

Es ist ebenfalls möglich, als Substrate nicht nur makroskopische Substrate einzusetzen, sondern auch andere Reagenzien, welche Aminogruppen und/oder Hydroxylgruppen enthalten, etwa Peptide, andere Wirkstoffe und/oder Zellen. Diese können untereinander, aber auch mit festen Trägern verbunden werden. Damit wird die Verwendung der erfindungsgemäßen Kombination zur Bindung von Peptiden und/oder Reagenzien, welche Aminogruppen und/oder Hydroxylgruppen enthalten, und/oder Zellen an Oberflächen, welche Aminosäuren enthalten oder mit Aminogruppen oder Hydroxylgruppen funktionalisiert sind, ermöglicht. Somit können z.B. Diagnostika mit einer kovalenten Verbindung zwischen einem Träger, z.B. einer ELISA-Platte, und einem diagnostisch verwendbaren Peptid hergestellt werden. Insbesondere bei sehr teuren Reagenzien führt dies zu einer wesentlichen Kostenreduktion. Die Empfindlichkeit von Nachweisreaktionen wird verbessert. Da intensivere Spülvorgänge eingesetzt werden können, wird die Spezifität der Nachweisreaktion erhöht. Genauso können reaktive Gruppen enthaltende Oberflächen unter Verwendung der erfindungsgemäßen Kombination mit Wirkstoffen, z.B. antibiotischen Wirkstoffen oder das Immunsystem hemmenden oder fördernden Substanzen, beschichtet werden.

Bevorzugt wird die erfindungsgemäße Kombination oder das erfindungsgemäße Verfahren zur Herstellung eines Medizinproduktes verwendet. Das Medizinprodukt ist besonders ein Produkt zum Verkleben von Organen, Geweben und/oder Knochen.

In einer Ausführungsform handelt es sich um ein Medizinprodukt zum Kleben von Weichgewebe. Dazu zählen z.B. Organe, Blutgefäße oder parenchymales Gewebe. Eine besonders bevorzugte Anwendung findet sich bei dem Verkleben von Wundrändern. Besonders ist es auch zum Kleben von Rupturen verschiedener Organe, wie Leber, Niere oder Milz, geeignet.

Auch die Versorgung von chirurgisch gesetzten Wunden, z.B. nach der Entfernung von Tumoren, ist mit dem erfindungsgemäßen Medizinprodukt möglich. Für den Wundverschluß z.B. bei großflächigen Sickerblutungen eignet sich insbesondere ein Produkt, bei dem als Lösungsmittel, wie im Detail oben beschrieben, eine Mischung von DMSO und Wasser eingesetzt wird.

Die Vorteile der erfindungsgemäßen Kombination ermöglichen die Anwendung in besonders schwierigen Fällen, insbesondere auch bei der Versorgung von chronischen Wunden. Bei solchen Wunden entsteht nach der notwendigen chirurgischen Versorgung eine starke Zugspannung, die die Wundheilung erschwert. Die hohe Festigkeit des erfindungsgemäßen Wundverschlusses ermöglicht auch in diesen Fällen eine feste Abdichtung und verhindert Wundinfektionen. Nach Abschluss der Vernetzungsreaktion können auf den so verschlossenen Wunden Kompressiv-Bandagen angewandt werden, um die Ödembildung zu verhindern.

Die Kleber können zur Fixierung von Implantaten, z.B. Gefäßprothesen, bioabbaubaren Implantaten, Kathetern, Stents und anderer Materialien genutzt werden. Eine Möglichkeit ist z.B. die Fixierung eines Herniennetzes an der Bauchwand. Bei einer vorteilhaften Ausführungsform werden in das zur Fixierung von Implantaten vorgesehene Klebersystem pharmazeutische Wirkstoffe eingelagert, um so Infektionen zu verhindern, unerwünschtes Zellwachstum zu unterbinden und Heilung zu beschleunigen. Mit Hilfe der eingelagerten pharmazeutischen Wirkstoffe können Resorption und Heilung aufeinander abgestimmt werden.

Eine vorteilhafte Anwendung dieser Kleber ist die flexible, über einen gewissen Zeitraum resorbierbare Abdichtung von Anastomosen und Patchen an Gefäßen bzw. auf Hohlorganen. Die Kleber sind auch für die Fixierung von Drug-Delivery Devices und von porösen Trägerstrukturen bzw. Membranen für den potentiellen Einsatz in der regenerativen Medizin (Tissue Engineering) geeignet.

Weitere erfindungsgemäße Verwendungen sind:
- als Mittel zur Ersten Hilfe nach Unfällen
- zur Wundsanierung bei infizierten Wunden
- in der plastischen, rekonstruktiven und/oder kosmetischen Chirurgie, insbesondere zur Verhinderung der Narbenbildung, die beim Nähen von Wunden induziert wird
- Verschluß und Abdichtung von Flüssigkeit- und Luftleckagen z. B. Abdichtung von Stichkanalblutungen in der Gefäßchirurgie, bei arteriellen Bypassoperationen oder zur Abdichtung von Lungenleckagen in der Thoraxchirurgie.

Überraschend wurde gefunden, dass die entwickelten Medizinprodukte auch hervorragend zum Kleben von Hartgewebe, z.B. zur Knochenklebung verwendet werden können. Für das Kleben von Knochen sowie für die schnelle Integration von Implantaten, insbesondere im Bereich der Knochen, ist die Akzeptanz des implantierten Materials durch die Knochenzellen von fundamentaler Bedeutung. Dies trifft natürlich auch auf die verwendeten Kleber zu. Durch die schnelle Besiedlung mit Osteoblasten und deren anschließender Ausreifung wird die Knochenneubildung gefördert und der Heilungsprozess verkürzt. Implantate werden daher bevorzugt mit mikrostrukturierten Oberflächen gefertigt. Der erfindungsgemäße Gewebekleber erhält diese Struktur und unterstützt die Knochenneubildung. Für die Produktion von Kollagen, das für die Knochenheilung unentbehrlich ist, sind die Aminosäuren Lysin und Prolin notwendig. Lysin ist an der Klebestelle in großem Umfang enthalten. Daraus resultiert eine positive Beeinflussung der Knochenbildung. Die gebildete extrazelluläre Matrix wird über Integrine, wie z.B. α1β1 oder α3β1, anhand der RGD-Sequenz (Arg-Gly-Asp) erkannt. Dadurch wird eine Signalkaskade in Gang gesetzt, die in der Zelle Veränderungen am Zytoskelett auslöst und zwischen Proliferationsstadium und Differenzierungsstadium umschaltet. Die gebildete Proteinmatrix dient außerdem der Einlagerung von Calciumsalzen.

Das ermöglicht die Anwendung bei komplizierten Brüchen und die Einfügung von Knochensplittern. Gerade bei der Operation komplizierter Brüche sind die Vorteile der erfindungsgemäßen hohen Klebekraft von großer Bedeutung. Das erfindungsgemäße Medizinprodukt dient daher bevorzugt zum Fixieren von Trümmerfrakturen z.B. im Gesichtsschädelbereich oder der Extremitäten, insbesondere bei Radiusköpfchenfraktur.

Ferner ist die erfindungsgemäße Kombination aufgrund der hohen Zugfestigkeit auch besonders zur Herstellung von Medizinprodukten zur Fixierung von Muskeln, Bändern und Sehnen an Knochen geeignet. In dieser Ausführungsform ist die Verwendung eines Katalysators der Reaktion zwischen Peptid und Oligolacton besonders empfehlenswert, um eine schnelle Aushärtung zu erreichen.

Eine weitere Anwendungsmöglichkeit findet sich im Bereich der Zahnmedizin, z.B. bei der Klebung von Inlays und Kronen oder bei Aufbaufüllungen. Auch die elastische Fixierung von Wurzelstiften mit dem erfindungsgemäßen Medizinprodukt anstelle von klassischem "Sealer" auf Basis von Methylmethacrylaten oder Glasionomer-Zementen ist möglich.

Die Viskosität und Fließfähigkeit der verwendeten Kombination kann, abhängig z.B. von der Länge der zu fixierenden Wunde und der Wundspalttiefe bzw. den zu verklebenden Substraten angepaßt werden. Neben der Länge der verwendeten Peptide und Oligolactone hat auch die Menge an Lösungsmittel einen Einfluß auf diese Parameter. Auch Zusatzstoffe wie Thixotropiemittel, z.B. nanodisperse Calciumphosphate (z.B. beta-Tricalciumphosphat (beta-TCP)) oder nanodisperse Kieselsäuren können zur Anpassung von Viskosität und Fließfähigkeit verwendet werden. Typischerweise wird zum Kleben von Hartgewebe ein Medizinprodukt mit höherer Viskosität verwendet als zum Kleben von Weichgewebe. Auch aus biologischen und mechanischen Gründen ist es insbesondere für die Verwendung als Hartgewebekleber vorteilhaft, wenn die erfindungsgemäße Kombination zusätzlich zu den oben im Detail beschriebenen Komponenten Calciumphosphat-Pulver umfasst, deren Anteil auf die gewünschte Anwendung abgestimmt werden kann.

Die Erfindung betrifft in einem weiteren Aspekt eine wie oben beschriebene erfindungsgemäße Kombination oder ein wie oben beschriebenes erfindungsgemäßes Medizinprodukt zur Verwendung in einem Verfahren zum Kleben von Organen, Geweben, Zahnkompartimenten und/oder Knochen, oder in einem Verfahren zum Verkleben von Wundrändern, oder in einem Verfahren zum Verschluss von Wunden oder Leckagen oder in einem Verfahren zum Kleben von Hart- oder Weichgewebe.

Die Erfindung soll in der Folge anhand von Ausführungsbeispielen erläutert werden, ohne auf diese Beispiele beschränkt zu sein.

### Legende

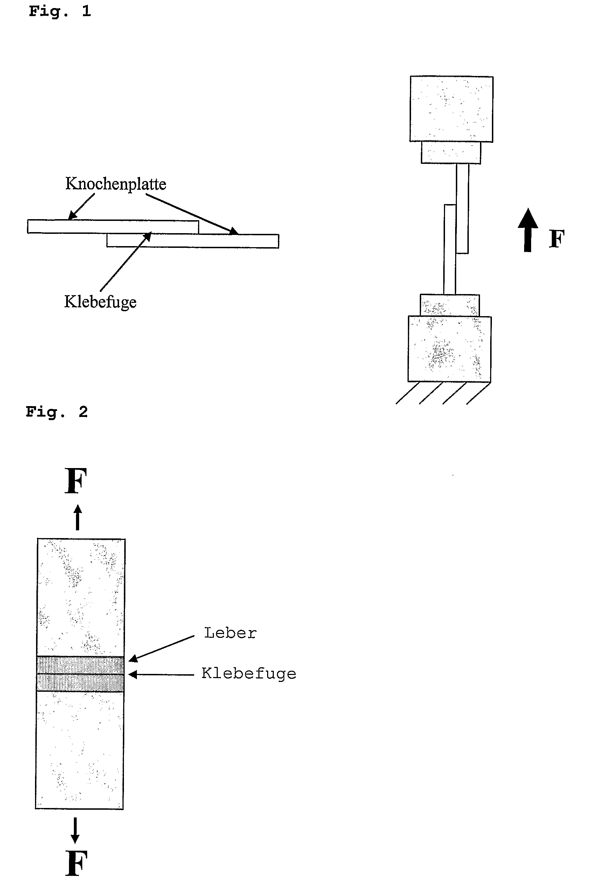

- Fig. 1: Anordnung zur Untersuchung der Klebfestigket bei Hartgewebeklebung
- Fig. 2:: Anordnung zur Untersuchung der Klebfestigkeit bei Weichgewebeklebung

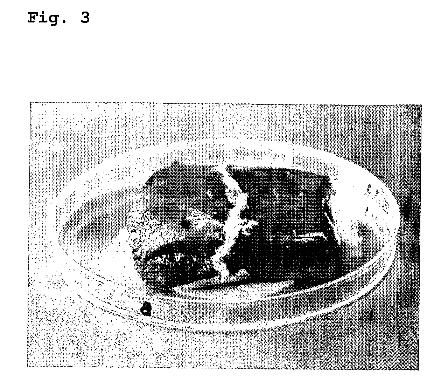

- Fig. 3:: Verkleben von Schweineleber als Weichgewebemodell

### Beispiele

### Beispiel 1a: Herstellung der Kleberpräpolymere und Durchführung der Klebereaktion für die Hartgewebeklebung durch manuelle Mischung der Klebekomponenten

Der entwickelte Kleber resultiert aus einer Mischung der einzelnen Komponenten, unter anderem des Oligopeptids 1 (Aminosäuresequenz aus Lysin und Tyrosin), wobei bevorzugt ein Oligopeptid n=5 bis n=10 verwendet wurde.

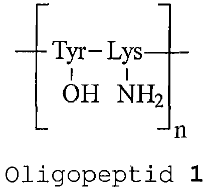

Ethylenglycol-Oligolactid (1/2, d.h. Umsetzung von 1 Teil Ethylenglykol mit 2 Teilen Lactid bei der ringöffnenden Polymerisation, n=1) wurde mit Hexamethylendiisocyanat (HDI) umgesetzt (siehe Reaktionsschema 2) und es resultierte aus der Reaktion EOL-NCO (Ethylenglycol-Oligolactid mit endständigen Isocyanatgruppen), das in den weiteren beschriebenen Versuchen verwendet wurde.

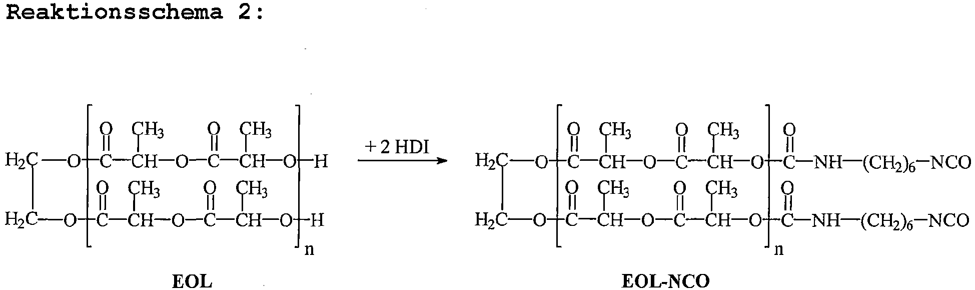

Die Klebereaktion wurde mit dem System EOL-NCO in Dimethylsulfoxid (DMSO) (Komponente 1) und Oligopeptid/1,4-Diaza[2.2.2]bicyclooctan (Dabco) als Katalysator in DMSO (Komponente 2) durchgeführt

### (Reaktionsschema 3).

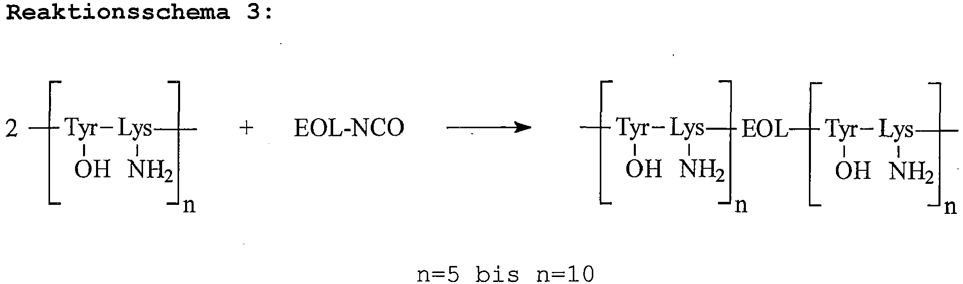

Dabei wird davon ausgegangen, dass die Reaktion des EOL-NCO mit den primären Aminogruppen und Hydroxylgruppen des Peptids erfolgt. Die Struktur des Polymerisats ist daher nur schematisch dargestellt.

Es wurde folgendes Mischungsverhältnis geprüft: EOL-NCO/Oligopeptid 4,7/1 (w/w) und EOL-NCO/Dabco 12,5/1 (w/w).

Um die Reaktion der einzelnen Komponenten schon beim manuellen Mischvorgang zu vermeiden, erfolgte ein zügiges Auftragen der Substanz auf das Hartgewebe.

Bei der mechanischen Testung mittels Zugmaschine wurde eine Klebfestigkeit von 36 mN/mm² ermittelt.

### Beispiel 1b: Durchführung der Klebereaktion für die Hartgewebeklebung ohne manuellen Mischungsvorgang

Da die Reaktion der Komponenten ziemlich schnell erfolgte, wurde versucht, die Komponenten ohne vorherigen Mischvorgang im oben beschriebenen Mengenverhältnis auf das Hartgewebe aufzutragen und zur Reaktion zu bringen. Das Vermischen der Komponenten durch das Zusammenbringen der Gewebekontaktflächen und die Reaktion der Komponenten erfolgten nun direkt auf dem Hartgewebe. Die Klebereaktion wurde mit dem System EOL-NCO in DMSO (Komponente 1) und Oligopeptid/Dabco als Katalysator in DMSO (Komponente 2) durchgeführt und war nach einigen Minuten abgeschlossen. Diese Modifizierung führte zu einer guten Haftfestigkeit. Hierbei wurde mittels Zugmaschine eine Klebfestigkeit von 88 mN/mm² ermittelt.

### Beispiel 1c: Durchführung der Klebereaktion ohne Katalysator für die Hartgewebeklebung durch manuelle Mischung der Klebekomponenten

Zur Beurteilung und Anpassung der Geschwindigkeit der Klebereaktion wurden außerdem Klebereaktionen ohne Katalysator untersucht. Deshalb wurde die Klebereaktion beispielsweise mit dem System EOL-NCO in DMSO (Komponente 1) und Oligopeptid in DMSO (Komponente 2) an Hartgewebe in den oben beschriebenen Mischungsverhältnissen durchgeführt.

Für die Ermittlung der Klebfugenendfestigkeit war die Bestimmung des 24 h-Wertes sinnvoll. Dabei stellte sich unerwarteterweise heraus, dass es nach diesem Zeitraum zu einer deutlichen Verbesserung der Haftfestigkeit der Klebepartner kam. Es resultierte eine Klebendfestigkeit (24 h) von 356 mN/mm².

### Beispiel 1d: Durchführung der Klebereaktion für die Weich- und Hartgewebeklebung durch Mischung der Klebekomponenten EOL-NCO/GOL/Dabco

Für die Untersuchungen wurde das System EOL-NCO in DMSO (Komponente 1), GOL (1/0,5, d.h. Umsetzung von 1 Teil Glycerol mit 0,5 Teilen Lactid bei der ringöffnenden Polymerisation) als Kettenverlängerer / Triethylamin bzw. Dabco als Katalysatoren in DMSO (Komponente 2) mitgeführt.

Das System EOL-NCO/DMSO (Komponente 1) und GOL/Dabco/DMSO (Komponente 2) wurde dann hinsichtlich Polymerisation und Haftung auf Weichgewebe getestet. Es wurden folgende Mischungsverhältnisse geprüft: EOL-NCO/GOL 1/1, 1,5/1, 2/1 und 4/1 (n/n, d.h. Molverhältnisse) sowie EOL-NCO/Dabco 20/1, 25/1 (w/w). Bei diesen Klebereaktionen zeigte sich, dass die Polymerisation in allen Fällen in ausreichender Geschwindigkeit zu festen Polyurethanen verlief, die Haftung zum Gewebe aber bei einem EOL-NCO/GOL-Mischungsverhältnis von 1,5/1 (n/n) und einem EOL-NCO/Dabco-Mischungsverhältnis von 25/1 (w/w) am höchsten war. Bei der mechanischen Testung der Klebefuge (Hartgewebeklebung) wurde eine Klebfestigkeit von 41 mN/mm² ermittelt.

### Beispiel 2: Prüfung der Festigkeit der Klebefuge an Hart- und Weichgewebe

Zur Prüfung der Festigkeit der Klebefuge im Zugversuch mussten die zu untersuchenden Gewebeteile vorher fixiert werden. Porcines Weichgewebe wurde mit Cyanacrylatkleber oder durch formschlüssige Verbindung an Probenhaltern aus Polymethylmethacrylat (PMMA) fixiert bzw. Knochenplatten aus Knochen bovinen Ursprungs wurden in mechanischen Klemmen eingespannt. Anschließend wurde die zu untersuchende Klebermischung (s. Bsp. 1) auf das Gewebe aufgetragen. Bei der Weichgewebeklebung wurde nach 10 Minuten die Festigkeit der Klebefuge unter Zugbeanspruchung an der Prüfmaschine Zwick BZ2.5/TN1S (Prüfgeschwindigkeit: 10 mm/min) ermittelt. Die mechanische Testung der Klebefuge nach Hartgewebeklebung erfolgte bei Zug-Scherbeanspruchung mit einer Prüfgeschwindigkeit von 5 mm/min.

### Beispiel 3: Applikation der Klebekomponenten mit Zweikammerspritzen

Um Erfolg versprechende Klebereaktionen aus den manuellen Anmischversuchen auf praxisübliche Bedingungen übertragen zu können, wurden diese Klebereaktionen mit Zweikammerspritzen inkl. Statikmischer der Fa. Mixpac Systems AG (Rotkreuz, Schweiz) wiederholt. Aufgrund der stöchiometrischen Verhältnisse von EOL-NCO zum Oligopeptid bzw. Kettenverlängerer kamen für die Untersuchungen vor allem Spritzen mit 4:1- und 10:1-Kammerverhältnis in Betracht.

In den durchgeführten Klebeuntersuchungen konnten hohe Haftfestigkeiten bei ausreichend schneller Vernetzung, beispielsweise unter Verwendung von 4:1-Spritzen, z.B. mit folgendem Klebersystem erreicht werden: EOL-NCO/DMSO (Komponente 1), GOL (1/0,5, d.h. Umsetzung von 1 Teil Glycerol mit 0,5 Teilen Lactid bei der ringöffnenden Polymerisation)/Dabco/DMSO/Wasser (Komponente 2), wobei das Mischungsverhältnis EOL-NCO/GOL 1,5/1 (n/n), EOL-NCO/Dabco 25/1 (w/w) vorlag. Für die Anwendung von 10:1-Spritzen wurde das oben genannte Klebersystem mit folgendem Mischungsverhältnis geprüft: EOL-NCO/GOL 10/1 (n/n), EOL-NCO/Dabco 25/1 (w/w). Bei Verwendung von DMSO/Wasser-Gemischen wurde eine besonders gute Haftfestigkeit erzielt, wenn nicht mehr als 12 % Wasser im Lösungsmittelgemisch vorhanden waren.

Entsprechende Bedingungen sind auch für das Kleben mit Peptid als Kettenverlängerer geeignet. Mit dem in diesem Beispiel beschriebenen Klebersystem konnten besonders gute Ergebnisse bei dem Kleben von Weichgewebe, z.B. von Lebergewebe, erzielt werden.

## Patentansprüche

1. Kombination, umfassend
a) ein Oligopeptid mit 2 bis 100 Aminosäuren Länge, das mindestens zwei reaktive Gruppen umfasst, ausgewählt aus einer Gruppe bestehend aus Hydroxyl und/oder Amino, und
b) ein reaktionsfähiges endständig funktionalisiertes Oligolacton, wobei das Oligolacton durch Umsetzung von 1 Teil Ethylenglykol mit 2 Teilen Lactid erhältlich ist, wobei der Anteil der Peptide an der Kombination 20-70 Gew.-%, bezogen auf die Gesamtmasse ohne Lösungsmittel, beträgt, und der Anteil der Oligolactone 30-80 Gew.-%, bezogen auf die Gesamtmasse ohne Lösungsmittel, beträgt,
wobei die Kombination ein medizinischer Kleber ist.

2. Kombination nach Anspruch 1, **dadurch gekennzeichnet, dass** etwa 50% der Aminosäuren des Oligopeptids Lysin und/oder etwa 50% der Aminosäuren des Oligopeptids Tyrosin sind.

3. Kombination nach den Ansprüchen 1 bis 2, **dadurch gekennzeichnet, dass** das Oligolacton ein Ethylenglycol-Oligolacton ist.

4. Kombination nach den Ansprüchen 1 bis 3, **dadurch gekennzeichnet, dass** das Oligolacton endständige Isocyanatgruppen aufweist.

5. Kombination nach den Ansprüchen 1 bis 4, **dadurch gekennzeichnet, dass** sie ferner
c) einen Katalysator für die Reaktion zwischen Oligopeptid und Oligolacton umfasst.

6. Kombination nach Anspruch 5, **dadurch gekennzeichnet, dass** der Katalysator ein stark basisches Amin, ein Amidin, ein tertiäres Amin und/oder ein Alkanolamin umfasst, wobei der Katalysator bevorzugt 1,4-Diaza[2.2.2]bicyclooctan (Dabco) ist.

7. Kombination nach den Ansprüchen 5 oder 6, umfassend
a) ein Oligopeptid, das mindestens eine Diaminosäure und/oder Hydroxylgruppe enthält,
b) ein mit Isocyanaten endständig funktionalisiertes Oligolacton und
c) einen Katalysator der Reaktion des Oligopeptids mit dem Oligolacton.

8. Kombination nach den Ansprüchen 1 bis 7, **dadurch gekennzeichnet, dass** sie zusätzlich
d) Kettenverlängerer zur Bulkpolymerisation, ausgewählt aus einer Gruppe umfassend Diole, Diamine und Oligolactone, ausgewählt aus einer Gruppe umfassend Ethylenglycol-Oligolactid, Ethylenglycololigoglycolid, Glycerololigolactid Glycerololigolactid-co-glycolid und Pentaerythritooligolactid, enthält.

9. Kombination nach den Ansprüchen 1 bis 8, **dadurch gekennzeichnet, dass** sie zusätzlich Kollagen und/oder Kollagenhydrolysate enthält.

10. Kombination nach den Ansprüchen 1 bis 9, **dadurch gekennzeichnet, dass** eine oder mehrere Komponenten in einem oder mehreren Lösungsmitteln gelöst ist/sind.

11. Kombination nach den Ansprüchen 1 bis 10, **dadurch gekennzeichnet, dass** die Komponenten a und b getrennt voneinander vorliegen.

12. Medizinprodukt zum Kleben von Organen, Gewebe, Zähnen und/oder Knochen, **dadurch gekennzeichnet, dass** es die Kombination nach den Ansprüchen 1 bis 11 umfasst.

13. Verbindung, **dadurch gekennzeichnet, dass** die Komponenten der Kombination nach den Ansprüchen 1 bis 10 miteinander vermischt und die Oligopeptide über die Oligolactone miteinander vernetzt sind.

14. In vitro Verfahren zum Kleben, **dadurch gekennzeichnet, dass** man die Komponenten der Kombination nach den Ansprüchen 1 bis 11 oder des Medizinprodukts nach Anspruch 12 miteinander und mit mindestens einem zu klebenden Substrat, das Hydroxylgruppen und/oder Aminogruppen aufweist, in Kontakt bringt, wobei das Verfahren bevorzugt in einem wässrigen Milieu durchgeführt wird.

15. In vitro Verfahren nach Anspruch 14, **dadurch gekennzeichnet, dass** mindestens eins der Substrate ein Organ, Gewebe, Zahnkompartiment und/öder Knochen ist.

16. Verfahren nach den Ansprüchen 14 oder 15, **dadurch gekennzeichnet, dass** die Verbindung der Substrate durch eine Vernetzung von Hydroxylgruppen und/oder Aminogruppen der Oligopeptide und der Substrate durch die Oligolactone erfolgt.

17. Verwendung der Kombination nach den Ansprüchen 1 bis 11 zur Bindung von Peptiden und/oder Reagenzien, welche Aminogruppen und/oder Hyxdroxylgruppen enthalten, und/oder von Zellen an Oberflächen, welche Aminosäuren enthalten oder mit Aminogruppen funktionalisiert sind.

18. Verwendung der Kombination nach den Ansprüchen 1 bis 11 oder des Verfahrens nach den Ansprüchen 14 bis 16 zur Herstellung eines Medizinprodukts.

19. Verwendung nach Anspruch 18, **dadurch gekennzeichnet, dass** das Medizinprodukt ein Produkt zum Kleben von Organen, Geweben, Zahnkompartimenten und/oder Knochen oder zum Verschluss von Wunden oder Leckagen oder ein Produkt zum Kleben von Hartgewebe oder ein Produkt zum Kleben von Weichgewebe oder ein Produkt zum Verkleben von Wundrändern oder ein Produkt zum Fixieren von Implantaten ist.

20. Kombination nach den Ansprüchen 1 bis 11 oder Medizinprodukt nach Anspruch 12 zur Verwendung in einem Verfahren zum Kleben von Organen, Geweben, Zahnkompartimenten und/oder Knochen, oder in einem Verfahren zum Verkleben von Wundrändern, oder in einem Verfahren zum Verschluss von Wunden oder Leckagen oder in einem Verfahren zum Kleben von Hart- oder Weichgewebe.

## Claims

1. Combination comprising
(a) an oligopeptide having a length of 2 to 100 amino acids comprising at least two reactive groups selected from the group consisting of hydroxyl and/or amino, and
(b) a reactive, terminal, functionalized oligolactone, wherein the oligolactone is obtainable by reacting one part ethylene glycol with two parts lactide, wherein the proportion of the peptides in the combination is 20-70% (w/w) based on the total mass without solvent, and the proportion of the oligolactones is 30-80% (w/w) based on the total mass without solvent,
wherein the combination is a medical adhesive.

2. Combination according to claim 1, wherein about 50% of the amino acids of the oligopeptides are lysine and/or about 50% of the amino acid of the oligopeptides are tyrosine.

3. Combination according to claims 1-2, wherein the oligolactone is an ethylene glycol oligolactone.

4. Combination according to claims 1-3, wherein the oligolactone has a terminal isocyanate group.

5. Combination according to claims 1-4, further comprising
(c) a catalyst for the reaction between oligopeptide and oligolactone.

6. Combination according to claim 5, wherein the catalyst comprises a strong basic amine, an amidine, a tertiary amine and/or an alkanol amine, wherein the catalyst preferably is 1,4-diazabicyclo[2.2.2]octane (Dabco).

7. Combination according to claim 5 or 6, comprising
(a) an oligopeptide comprising at least one diamino acid and/or hydroxyl group,
(b) a terminal oligolactone which has been functionalized with isocyanates, and
(c) a catalyst for the reaction of the oligopeptide with the oligolactone.

8. Combination according to claims 1-7, further comprising
(d) a chain extender for bulk polymerisation, selected from the group comprising diols, diamines and oligolactones, selected from the group consisting of ethylene glycol oligolactide, ethylene glycol oligoglycolide, glycerol oligolactide, glycerol oligolactide-co-glycolide and pentaerythritol oligolactide.

9. Combination according to claims 1-8, further comprising collagen and/or collagen hydrolysate.

10. Combination according to claims 1-9, wherein one or more components are dissolved in one or more solvent.

11. Combination according to claims 1-10, wherein components a and b occur separately from each other.

12. Medical product for gluing organs, tissues, teeth and/or bones, wherein said medical product comprises the combination according to claims 1-11.

13. Compound, **characterized in that** the components of the combination according to claims 1-10 are mixed with each other and the oligopeptides are cross-linked with each other via the oligolactones.

14. In vitro method for gluing comprising contacting the components of the combination of claims 1-11 or the medical product according to claim 12 with each other and with at least one substrate to be glued which comprises hydroxyl groups and/or amino groups, wherein said method is preferably conducted in an aqueous environment.

15. In vitro method according to claim 14, wherein at least one of the substrates is an organ, a tissue, a tooth compartment and/or a bone.

16. Method according to claims 14 or 15, wherein joining of the substrates occurs by crosslinking of hydroxyl groups and/or amino groups of the oligopeptides and the substrates via the oligolactones.

17. Use of the combination of claims 1-11 for binding peptides and/or reagents, which comprise amino groups and/or hydroxyl groups, and/or cells to surfaces, which comprise amino acids or are functionalized with amino groups.

18. Use of the combination of claims 1-11 or the method according to claims 14-16 for the preparation of a medical product.

19. Use according to claim 18, wherein the medical product is a product for gluing organs, tissues, tooth compartments and/or bones or for closing wounds or leakages, or a product for gluing of hard tissue, or a product for gluing of soft tissue, or a product for gluing of wound edges, or a product for the fixing of implants.

20. Combination according to claims 1-11 or medical product according to claim 12 for use in a method for gluing organs, tissues, tooth compartments and/or bones, or in a method for gluing of wound edges, or in a method for closing of wounds or leakages, or in a method for gluing of hard or soft tissues.

## Revendications

1. Combinaison comprenant :
a) un oligopeptide long de 2 à 100 acides aminés, qui comporte au moins deux groupes réactifs choisis dans l'ensemble constitué par des groupes hydroxyle et/ou amino,
b) et une oligolactone réactive fonctionnalisée en bouts de chaîne, laquelle oligolactone est accessible par réaction de 1 partie d'éthylèneglycol avec 2 parties de lactide,
étant entendu que la proportion de peptide dans la combinaison vaut de 20 à 70 %, en poids rapporté au poids total sans solvant, et que la proportion d'oligolactone vaut de 30 à 80 %, en poids rapporté au poids total sans solvant,
laquelle combinaison est un adhésif médical.

2. Combinaison conforme à la revendication 1, **caractérisée en ce qu'**environ 50 % des acides aminés de l'oligopeptide sont des résidus de lysine et/ou environ 50 % des acides aminés de l'oligopeptide sont des résidus de tyrosine.

3. Combinaison conforme à la revendication 1 ou 2, **caractérisée en ce que** l'oligolactone est une oligolactone dérivée d'éthylèneglycol.

4. Combinaison conforme à l'une des revendications 1 à 3, **caractérisée en ce que** l'oligolactone porte des groupes isocyanate terminaux.

5. Combinaison conforme à l'une des revendications 1 à 4, **caractérisée en ce qu'**elle comporte en outre
c) un catalyseur pour la réaction entre l'oligopeptide et l'oligolactone.

6. Combinaison conforme à la revendication 5, **caractérisée en ce que** le catalyseur comprend une amine fortement basique, une amidine, une amine tertiaire et/ou une alcanolamine, le catalyseur étant de préférence du 1,4-diaza[2.2.2]bicyclooctane (Dabco).

7. Combinaison conforme à la revendication 5 ou 6, comprenant :
a) un oligopeptide qui contient au moins un diamino-acide et/ou un groupe hydroxyle,
b) une oligolactone fonctionnalisée en bouts de chaîne avec des groupes isocyanate,
c) et un catalyseur de la réaction de l'oligopeptide avec l'oligolactone.

8. Combinaison conforme à l'une des revendications 1 à 7, **caractérisée en ce qu'**elle comporte en outre
d) un agent d'allongement de chaînes pour polymérisation en masse, choisi dans l'ensemble comprenant les diols, les diamines et les oligolactones choisies dans l'ensemble comprenant les suivantes : éthylèneglycol-oligolactide, éthylèneglycol-oligoglycolide, glycérol-oligolactide, glycérol-oligo(lactide-co-glycolide) et pentaérythritol-oligolactide.

9. Combinaison conforme à l'une des revendications 1 à 8, **caractérisée en ce qu'**elle comporte en outre du collagène et/ou un hydrolysat de collagène.

10. Combinaison conforme à l'une des revendications 1 à 9, **caractérisée en ce qu'**un ou plusieurs composant(s) est ou sont dissous dans un ou plusieurs solvant(s).

11. Combinaison conforme à l'une des revendications 1 à 10, **caractérisée en ce que** les composants (a) et (b) sont séparés l'un de l'autre.

12. Produit médical pour collage d'organes, de tissus, de dents et/ou d'os, **caractérisé en ce qu'**il comprend une combinaison conforme à l'une des revendications 1 à 11.

13. Opération de liaison, **caractérisée en ce qu'**on mélange les uns avec les autres les composants d'une combinaison conforme à l'une des revendications 1 à 10, et les oligopeptides sont réticulés les uns avec les autres par l'intermédiaire des oligolactones.

14. Procédé de collage in vitro, **caractérisé en ce que** l'on met les composants d'une combinaison conforme à l'une des revendications 1 à 11, ou d'un produit médical conforme à la revendication 12, en contact les uns avec les autres et avec au moins un substrat à coller, qui porte des groupes hydroxyle et/ou amino, lequel procédé est mis en oeuvre de préférence dans un milieu aqueux.

15. Procédé in vitro conforme à la revendication 14, **caractérisé en ce qu'**au moins l'un des substrats est un organe, un tissu, un compartiment de dent et/ou un os.

16. Procédé conforme à la revendication 14 ou 15, **caractérisé en ce que** la liaison des substrats est opérée par réticulation entre les groupes hydroxyle et/ou amino des oligopeptides et ceux des substrats, par l'intermédiaire des oligolactones.

17. Utilisation d'une combinaison conforme à l'une des revendications 1 à 11 pour attacher des peptides et/ou des réactifs comportant des groupes amino et/ou hydroxyle et/ou des cellules à des surfaces qui comportent des acides aminés ou qui sont fonctionnalisées au moyen de groupes amino.

18. Utilisation d'une combinaison conforme à l'une des revendications 1 à 11 ou d'un procédé conforme à l'une des revendications 14 à 16 pour la fabrication d'un produit médical.

19. Utilisation conforme à la revendication 18, **caractérisée en ce que** le produit médical est un produit pour collage d'organes, de tissus, de compartiments de dent et/ou d'os ou pour fermeture de plaies ou arrêt d'épanchements, ou un produit pour collage de tissus durs, un produit pour collage de tissus mous, un produit pour collage de bords de plaies, ou un produit pour fixation d'implants.

20. Combinaison conforme à l'une des revendications 1 à 11, ou produit médical conforme à la revendication 12, pour utilisation dans un procédé de collage d'organes, de tissus, de compartiments de dent et/ou d'os, ou dans un procédé de collage de bords de plaies, dans un procédé de fermeture de plaies ou d'arrêt d'épanchements, ou dans un procédé de collage de tissus durs ou de tissus mous.
